# EUROPEAN PATENT APPLICATION

(11) **EP 4 722 727 A1**
(43) Date of publication of application: **08.04.2026**
(21) Application number: 24815307.4
(22) Date of filing: 21.05.2024
(51) Int. Cl.: G01N 35/00

(54) **AUTOMATIC ANALYSIS DEVICE AND METHOD FOR ALLOCATING ANALYSIS TANK**

(30) Priority: 30.05.2023 JP 2023088603
(71) Applicant: HITACHI HIGH-TECH CORPORATION, Tokyo 105-6409 (JP)
(72) Inventor: NISHINO Kanoko, Tokyo 105-6409 (JP)
(74) Representative: MERH-IP Matias Erny Reichl Hoffmann Patentanwälte PartG mbB
(86) International application number: PCT/JP2024/018709
(87) International publication number: WO 2024/247823

(57) **Abstract**

This automatic analysis device calculates, for each of a plurality of analysis tanks 106, a target number of uses of the analysis tank per predetermined period of time on the basis of the number of remaining executable measurements of an ion selective electrode 112 and a remaining period of time up to an expiration date thereof, and when there are a plurality of analysis tanks usable for an analysis request, determines a ratio of usage for each analysis tank on the basis of a ratio of the target number of uses of each of the plurality of analysis tanks usable for the analysis request. This allows as many of the number of remaining executable measurements as possible to be used in the automatic analysis device comprising the plurality of analysis tanks before the expiration date of the ion selective electrode.

## Description

### Technical Field

The present invention relates to an automatic analysis device including a plurality of analysis tanks for measuring the electrolyte concentration of a sample, and a method for allocating the analysis tank.

### Background Art

An electrolyte measurement is conducted using ion selective electrodes (sodium(Na)/potassium(K)/chloride(Cl)). An ion selective electrode is one of consumables in electrolyte measurement, and each ion selective electrode has an expiration date and the number of remaining possible measurements. However, the expiration date and the number of remaining possible measurements of the ion selective electrode are hardly managed. The ion selective electrode having expired or the ion selective electrode with no remaining possible measurements cannot be used for electrolyte measurement. Thus, for example, even when the number of remaining possible measurements is large, the ion selective electrode having expired cannot be used for electrolyte measurement.

Patent Literature 1 discloses an electrolyte analysis device that properly exchanges consumables by allocating an analysis tank to be used for analysis, the analysis tank being allocated using the number of remaining possible measurements or the expiration date. Specifically, in response to an analysis request to the electrolyte analysis device, a control computer first determines whether the number of measurement requests to be processed in a predetermined time is greater than or equal to the maximum processing capacity or is less than the maximum processing capacity. When the number of measurement requests is less than the maximum processing capacity, the analysis tank is allocated such that the analysis tank with the largest number of remaining possible measurements of ion selective electrode ion selective electrode is used with priority. At this point, it is assumed that the analysis tank can be allocated using the expiration date of the ion selective electrode in addition to or instead of the number of remaining possible measurements. In this case, the analysis tank to be preferentially used is determined on the basis of two criteria: the number of remaining possible measurements and the expiration date of the ion selective electrode. Furthermore, a user can select the analysis tank to be preferentially used, on the basis of the number of remaining possible measurements and the expiration date of the ion selective electrode.

In this way, an analysis operation is performed to preferentially keep the processing capacity when the analysis request status is close to the maximum processing capacity, whereas in the case of an intermittent request status, the analysis tank with a large number of remaining possible measurements of the ion selective electrode is preferentially used, so that the plurality of analysis tanks can have an equal number of remaining possible measurements. This allows the user to exchange the ion selective electrodes of the plurality of analysis tanks at the same timing, so that the user can exchange consumables with proper frequency.

### Citation List

### Patent Literature

Patent Literature 1: International Publication WO No. 2022/014096

### Summary of Invention

### Technical Problem

In Patent Literature 1, the ion selective electrodes of the analysis tanks are exchanged at the same timing, thereby reducing the number of exchanges of consumables. However, since the plurality of analysis tanks have an equal number of remaining possible measurements, the ion selective electrode may expire and need replacement depending on the analysis request status even if the ion selective electrode has a large number of remaining possible measurements. It is desirable to use the remaining possible measurements of the ion selective electrode as a consumable as many as possible before the expiration date.

An object of the present invention is to consume remaining possible measurements as many as possible before the expiration date of the ion selective electrode in an automatic analysis device including the plurality of analysis tanks. Solution to Problem

An automatic analysis device according to an embodiment of the present invention includes: the plurality of analysis tanks, each including the ion selective electrode for measuring an electrolyte concentration of a sample, and a control computer that allocates the analysis tank to be used for an analysis request. The control computer calculates, for each of the analysis tanks, the target number of times the analysis tank is to be used in a predetermined period on the basis of the number of remaining possible measurements of the ion selective electrode and a remaining period before an expiration date, and determines, when the plurality of analysis tanks are usable for the analysis request, the use ratio of the analysis tank on the basis of a ratio of the target number of times of the analysis tanks usable for the analysis request.

### Advantageous Effects of Invention

In the automatic analysis device including the plurality of analysis tanks, the number of remaining possible measurements can be consumed before the expiration date of the ion selective electrode. Problems, configurations, and effects other than those described above will be clarified by the description of the following embodiment.

### Brief Description of Drawings

[Fig. 1] Fig. 1 is a schematic diagram illustrating a configuration example of an automatic analysis device.
[Fig. 2] Fig. 2 is a flowchart showing the overall processing of allocation of an analysis tank.
[Fig. 3] Fig. 3 is a flowchart showing processing for determining the analysis tank to be used.
[Fig. 4] Fig. 4 is a flowchart showing processing for determining the use ratios of the plurality of analysis tanks determined as usable analysis tanks.
[Fig. 5] Fig. 5 shows an example of the analysis tank selection screen for specifying the analysis tank to be used.
[Fig. 6] Fig. 6 shows an example of an analysis tank priority use setting screen that enables/disables the function of the present example.

### Description of Embodiments

An embodiment of an automatic analysis device will be described below in accordance with the accompanying drawings. The following embodiment will describe an example of the automatic analysis device including an electrolyte analysis device (analysis module) having a plurality of analysis tanks. The device configuration is not limited thereto. The automatic analysis device may include a plurality of electrolyte analysis devices, each having the single analysis tank.

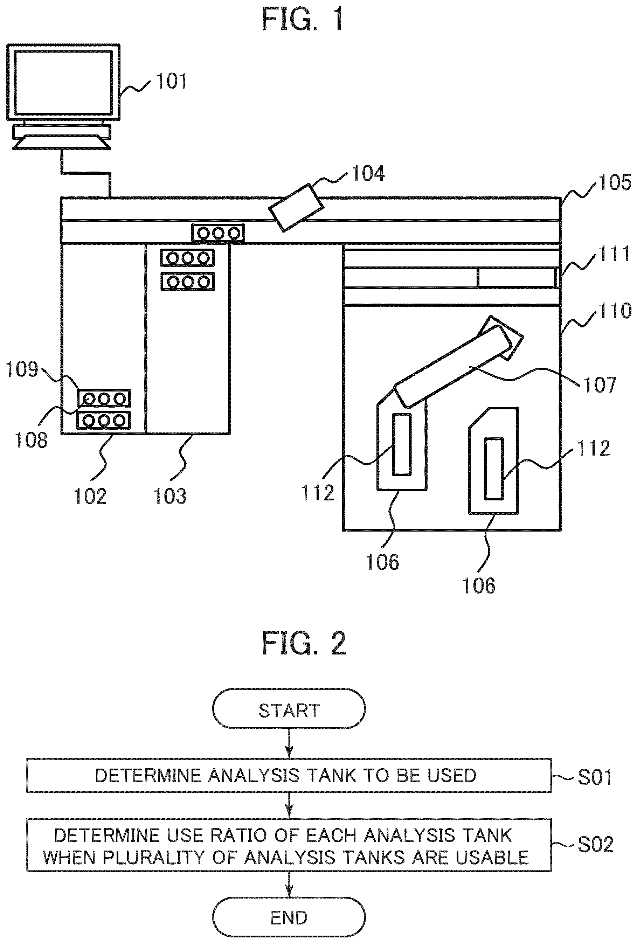

Referring to Fig. 1, a configuration example of the automatic analysis device will be described below. The automatic analysis device includes a control computer 101, a sample loading part 102, a sample collection part 103, an ID reader 104, a conveyor line 105, analysis tanks 106, a dispensing mechanism 107, and an analysis module (electrolyte analysis device) 110. The analysis module 110 includes the plurality of (in this example, two) analysis tanks 106.

On the sample loading part 102, carriers 109 loaded with sample containers 108 are loaded, the sample container 108 storing a sample such as blood or urine that is collected from a subject. The loaded carrier 109 is conveyed to the ID reader 104 by the conveyor line 105. The ID reader 104 is a device that reads a carrier ID attached to the carrier 109 and a sample ID attached to the sample container 108 loaded on the carrier 109. The ID reader 104 is, for example, a bar code reader or an RFID reader. On the basis of information indicated by the carrier ID and the sample ID, the sample is associated with an analysis item requested from a user in advance.

After the sample and the requested analysis item are associated with each other by the ID reader 104, the carrier 109 is conveyed to the analysis module 110 through the conveyor line 105. The analysis module 110 receives the carrier 109 conveyed through the conveyor line 105 and conveys the carrier 109 to the dispensing position by using an intra-analysis unit conveyor line 111. Thereafter, the sample stored in the sample container 108 is sucked by using the dispensing mechanism 107 on the basis of the requested analysis item, and is dispensed to one of the analysis tanks 106. The dispensed sample is measured by the detector of the analysis tank 106.

After the completion of dispensing, the carrier 109 is delivered to the conveyor line 105 by using the intra-analysis unit conveyor line 111. Thereafter, the carrier 109 is collected to the sample collection part 103 through the conveyor line 105.

The analysis tanks 106 each include an ion selective electrode 112 to perform electrolyte analysis. For the ion selective electrode 112, the number of remaining possible measurements and an expiration date are set. The automatic analysis device of the present embodiment allocates the analysis tank 106 to be used for the analysis request, by using the target number of times that is determined on the basis of the number of remaining possible measurements and the expiration date of the ion selective electrode 112. As described above, three kinds of ion selective electrodes made of sodium, potassium, and chloride are used. Therefore, when three kinds of ion selective electrodes are different in the number of remaining possible measurements and the expiration date, in the following flow of selecting the analysis tank, it is desirable to use the number of remaining possible measurements and the expiration date of the ion selective electrode having the shortest remaining period before the expiration date. However, in the configuration of the electrolyte analysis device, all the ion selective electrodes are used for each measurement regardless of the content of the analysis item. Thus, three kinds of ion selective electrodes are frequently exchanged at the same time. In the automatic analysis device being operated thus, the number of remaining possible measurements and the expiration date of any ion selective electrode may be used for allocating the analysis tank.

Fig. 2 is a flowchart showing the overall processing of allocation of the analysis tank. The flowchart is implemented by the control computer 101.

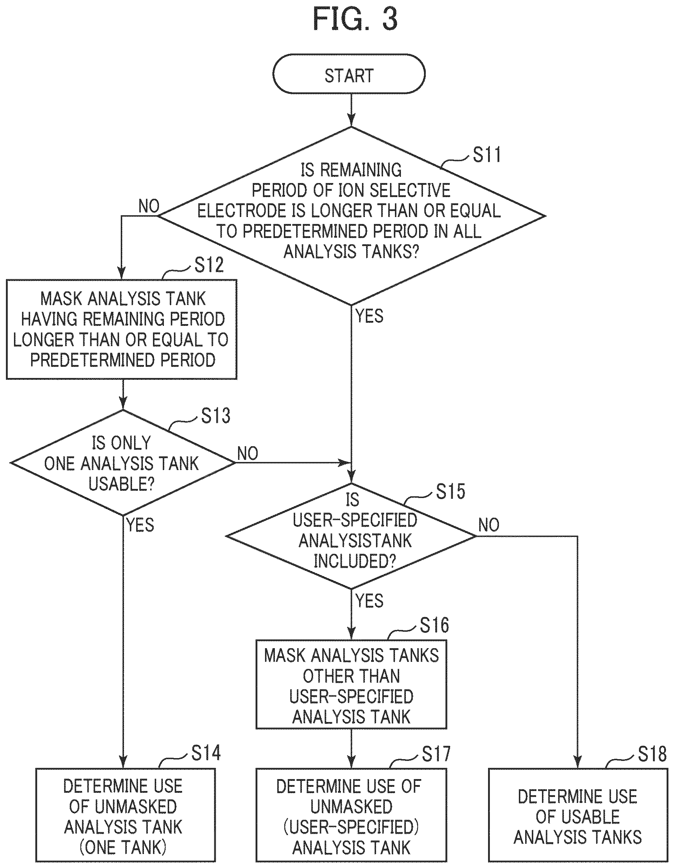

First, the control computer 101 determines the analysis tank 106 to be used for analysis (S01). Thus, the analysis tank to be preferentially used can be used for analysis, thereby reducing the number of remaining possible measurements of the ion selective electrode before the expiration date. Fig. 3 shows the detail of Step S01. Subsequently, if the plurality of analysis tanks are determined as usable tanks in Step S01, the control computer 101 determines the use ratios of the analysis tanks 106 (S02).

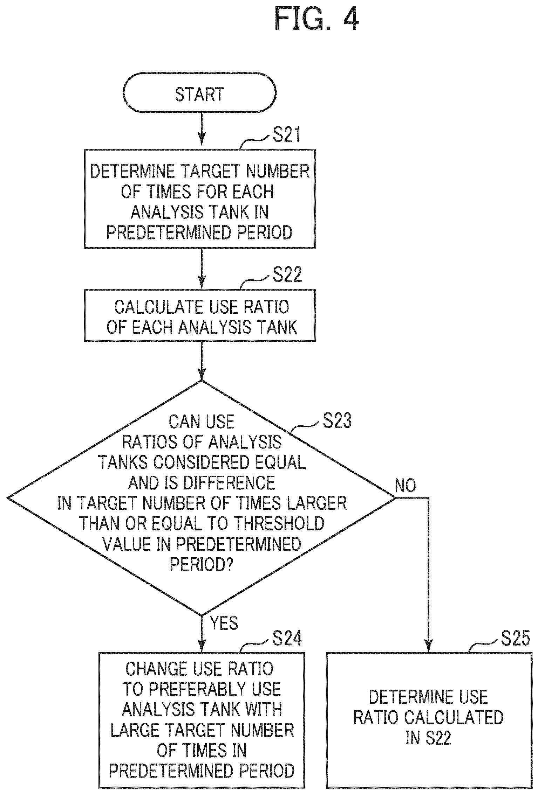

Fig. 4 shows the detail of Step S02.

Referring to Fig. 3, the detail of processing (Step S01) for determining the analysis tank to be used will be described below.

Step S11: It is determined whether or not a remaining period before the expiration date of the ion selective electrode is longer than or equal to a predetermined period in all the analysis tanks 106 provided in the automatic analysis device. The predetermined period is not particularly specified but will be designated as 24 hours in the following description. In this case, the control computer 101 determines whether or not the remaining period of the ion selective electrode 112 of the analysis tank 106 is longer than or equal to 24 hours. When the remaining period of the ion selective electrode 112 is 24 hours or longer in all the analysis tanks 106, all the analysis tanks 106 are determined as usable analysis tanks, and then the process advances to Step S15. In contrast, in the presence of the analysis tank 106 in which the ion selective electrode 112 expires in a period shorter than 24 hours, the process advances to Step S12.

Step S12: The control computer 101 masks the analysis tank 106 in which the ion selective electrode 112 has a remaining period longer than or equal to a predetermined period. In this case, the control computer 101 masks the analysis tank 106 in which the remaining period of the ion selective electrode 112 is at least 24 hours. Therefore, among the analysis tanks provided in the automatic analysis device, the analysis tank 106 including the ion selective electrode 112 with a remaining period longer than or equal to the predetermined period is excluded from the usable analysis tanks.

Step S13: It is determined whether only one of the analysis tanks 106 is usable or not. If only one of the analysis tanks 106 is usable, the analysis tanks 106 to be used for measurement cannot be narrowed down any more. Thus, the process advances to Step S14. When the plurality of analysis tanks 106 can be used in Step S13, the analysis tanks 106 including the ion selective electrodes 112 with remaining periods shorter than the predetermined period are included as the usable analysis tanks, and then the process advances to Step S15.

Step S14: It is determined that the unmasked analysis tank (one tank) is to be used.

Step S15: The control computer 101 determines whether the usable analysis tanks 106 include the user-specified analysis tank.

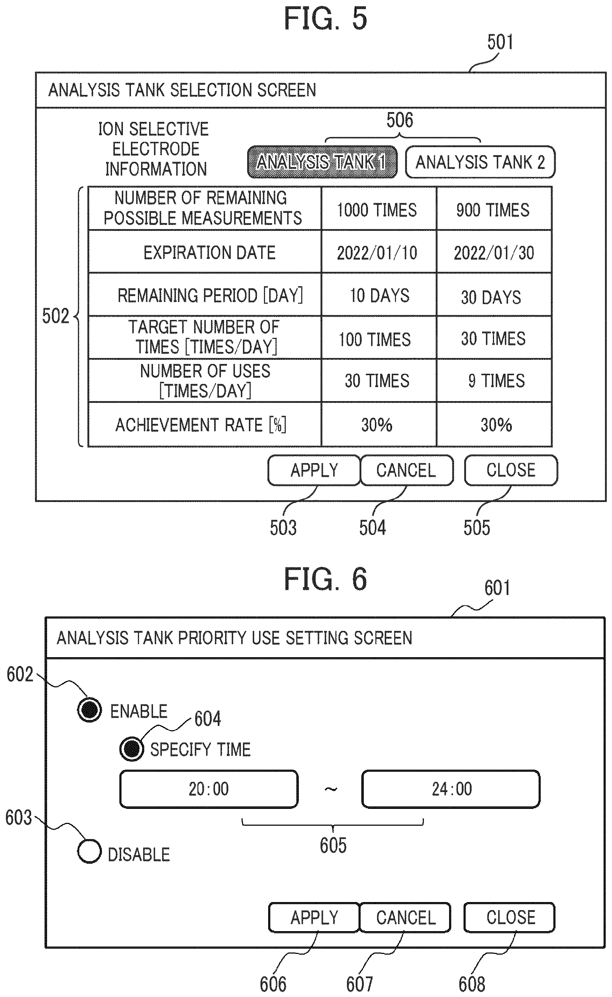

At any timing, the analysis tank selection screen 501 shown in Fig. 5 allows a user to confirm the states of the analysis tanks 106 at that time in an ion-selective-electrode information display field 502 and select the analysis tank 106 to be used.

Fig. 5 is a screen example in which the automatic analysis device includes two analysis tanks: an analysis tank 1 and an analysis tank 2. Information about the analysis tanks is displayed in the ion-selective-electrode information display field 502. The ion-selective-electrode information display field 502 includes at least the number of remaining possible measurements and the expiration date of the analysis tank 106. In this example, a remaining period before the expiration date, the target number of times that is obtained by dividing the number of remaining possible measurements by the remaining period, and the number of uses and an achievement rate (the number of uses/the target number of times) at the time of display of the analysis tank selection screen 501 are shown to facilitate determination. The user can specify the analysis tank 106 by pressing an analysis tank selection button 506 of one of the analysis tanks on the basis of information of the ion-selective-electrode information display field 502 and pressing an application button 503 or a cancel button 504. From the ion-selective-electrode information display field 502, the user can securely confirm the state of the ion selective electrode 112 that is unlikely to expire. Moreover, only the analysis tank 106 to be preferentially used can be used by specifying the analysis tank 106. When the analysis tank selection screen 501 is closed, a close button 505 is pressed.

In the determination of Step S15, the latest user selection is used. However, for example, when the user selection is made after a predetermined expiration date, the user selection may be rendered ineffective. In the absence of the user-specified analysis tank or an ineffective user specification, the process advances to Step S18. In the presence of the user-specified analysis tank, the process advances to Step S16.

Step S16: The control computer 101 masks the analysis tanks 106 other than the user-specified analysis tank 106. Therefore, among the analysis tanks provided in the automatic analysis device, the analysis tanks 106 other than the user-specified analysis tank 106 are excluded from the usable analysis tanks.

Step S17: The use of the unmasked analysis tank, that is, the user-specified analysis tank is determined.

Step S18: The use of the usable analysis tank 106 is determined. In this case, the plurality of analysis tanks are usable.

In the example of Fig. 3, the analysis tank to be preferentially used is limited on two criteria: the expiration date and the user specification. The analysis tank is limited only by one of the criteria, or Step S01 may be omitted. However, by performing Step S01, the analysis tank to be used can be limited to the analysis tank to be preferentially used, for example, the user-specified analysis tank or the analysis tank that is about to expire.

In the processing of Step S01, a plurality of analysis tanks may be determined as usable analysis tanks (Steps S17 and S18). In such a case, the control computer 101 performs Step S02. When only one analysis tank is usable (Step S14), the execution of Step S02 is omitted. Referring to Fig. 4, the detail of processing (Step S02) for determining the use ratios of the plurality of analysis tanks determined as usable analysis tanks. In Step S02, the use ratio of each of the analysis tanks 106 is calculated on the basis of the number of remaining possible measurements of the ion selective electrode 112 and a remaining period before the expiration date.

Step S21: The control computer 101 calculates, for each of the analysis tanks, the target number of times the analysis tank is to be used in a predetermined period. The predetermined period is not particularly specified but will be designated as one day in the following description. First, from the expiration date of the ion selective electrode 112 in each of the analysis tanks and the current time, a remaining period before the expiration date is calculated. When the predetermined period is one day, the remaining number of days is treated as the remaining period. The target number of times is the number of times that is obtained by dividing the number of remaining possible measurements of the ion selective electrode 112 by the remaining number of days. In this way, the target number of times may be calculated as a simple average or may be calculated by assigning a weight, e.g., a day of a week.

Step S22: The control computer 101 determines the use ratios of the analysis tanks 106. A method for determining the use ratio is determined on the basis of the ratio of the target number of times in a predetermined period of each of the analysis tanks 106. For example, in the case of the two analysis tanks 106: the analysis tank 106 in which the target number of times is 100 and the analysis tank 106 in which the target number of times is 200 in a predetermined period, the ratio of the target number of times can be calculated as 1 to 2.

Step S23: In Step S23, it is determined whether the calculated use ratios of the analysis tanks can be considered equal and a difference in the target number of times that is calculated in Step S21 is larger than or equal to a threshold value in a predetermined period. The step is processing for eliminating a difference in the target number of times between the plurality of analysis tanks in the predetermined period even if the target number of times is nearly equal in the analysis tanks.

For example, in the case of the two analysis tanks 106: the analysis tank 106 in which the target number of times is 97 and the analysis tank 106 in which the target number of times is 103 in a predetermined period, in Step S22, the use ratio is assumed to be 1:1, that is, the use ratio is assumed to be equal in the two analysis tanks. However, a difference in the target number of times between the analysis tanks is six times in the predetermined period. Thus, a threshold value of a difference in the target number of times in the predetermined period is set in advance. In the case of a difference larger than or equal to the threshold value, the use ratio is changed to eliminate the difference.

Step S24: The process advances to this step when the conditions of Step S23 are satisfied. The use ratio is changed to preferentially use the analysis tank 106 with a large target number of times in the predetermined period. For example, in the case of the two analysis tanks 106: the analysis tank 106 in which the target number of times is 97 and the analysis tank 106 in which the target number of times is 103 in the predetermined period, when a threshold value of a difference in the target number of times is three times in the predetermined period, the use ratio is changed to 1:2. Thus, since no difference is made in use ratio between the analysis tanks 106, the use ratio being calculated in Step S21, a difference in the number of remaining possible measurements can be prevented from increasing.

Step S25: The process advances to this step when the conditions of Step S23 are not satisfied. The use ratio of each of the analysis tanks 106 is determined as the use ratio calculated in Step S22.

According to the foregoing description, a proper use ratio can be determined to use the remaining possible measurements of the ion selective electrode 112 before the expiration date.

Processing described in Figs. 2 to 4 is performed when the date changes or when the automatic analysis device starts for the first time on that day. Thereafter, when the control computer 101 determines the analysis tank 106 to be used in response to the analysis request, the analysis tanks excluded from the usable analysis tank are masked. Thus, the control computer 101 does not need to select the analysis tank 106 to be used in response to each analysis request. When the single analysis tank is selected to be used, the analysis tank is determined in a fixed manner, whereas when the plurality of analysis tanks are determined to be used, the analysis request may be allocated to the analysis tanks with the calculated use ratio. Also when the user changes the analysis tank specified from the analysis tank selection screen 501 (see Fig. 5), the processing in Figs. 2 to 4 is performed.

However, the present embodiment may suppress the processing capacity of the automatic analysis device (electrolyte analysis device) including the plurality of analysis tanks. Hence, it is also effective to enable switching between an analysis priority mode in which the throughput of the analysis request is improved by maximizing the number of analyses in a predetermined time without performing the function of the present embodiment and a consumable effective use mode in which the function of the present embodiment is performed to effectively use consumables.

For example, the user can switch the modes through an enable button 602 and a disable button 603 of an analysis tank priority use setting screen 601 shown in Fig. 6. Specifically, whether to enable or disable the function of the present embodiment is selected, and then the selection is set using an application button 606 and a cancel button 607. When the function is enabled by the enable button 602, whether to specify a time can be set by a time specification setting button 604. When a time for enabling the function is specified by the time specification setting button 604, the time for enabling the function can be set in specified-time input fields 605. When the analysis tank priority use setting screen 601 is closed, a close button 608 is pressed.

This allows the enable/disable setting of the function. The function can be selectively applied according to the analysis request status, thereby effectively using consumables without reducing the substantial throughput of the automatic analysis device.

The present invention is not limited to the foregoing embodiment and modifications and includes other various modifications. For example, the foregoing embodiment has been described in detail for the sake of clarity of the present invention. The present invention is not limited to an embodiment including all the foregoing configurations. For example, if the automatic analysis device includes the analysis tank in which the ion selective electrode 112 is about to expire, the present invention may have the function of masking other analysis tanks and the function of allowing a user to confirm the masked analysis tanks.

### List of Reference Signs

101: control computer, 102: sample loading part, 103: sample collection part, 104: ID reader, 105: conveyor line, 106: analysis tank, 107: dispensing mechanism, 108: sample container, 109: carrier, 110: analysis module, 111: intra-analysis unit conveyor line, 501: analysis tank selection screen, 502: ion-selective-electrode information display field, 503, 606: application button, 504, 607: cancel button, 505, 608: close button , 506: analysis tank selection button, 601: analysis tank priority use setting screen, 602: enable button, 603: disable button, 604: time specification setting button, 605: specified-time input field

## Claims

1. An automatic analysis device comprising:
a plurality of analysis tanks, each including an ion selective electrode for measuring an electrolyte concentration of a sample, and
a control computer that allocates the analysis tank to be used for an analysis request,
wherein the control computer calculates, for each of the analysis tanks, a target number of times the analysis tank is to be used in a predetermined period on a basis of the number of remaining possible measurements of the ion selective electrode and a remaining period before an expiration date, and determines, when the plurality of analysis tanks are usable for the analysis request, a use ratio of the analysis tank on a basis of a ratio of the target number of times of the analysis tanks usable for the analysis request.

2. The automatic analysis device according to claim 1,
wherein each of the analysis tanks includes a plural kinds of ion selective electrodes, and
the control computer calculates the target number of times by using the number of remaining possible measurements and the expiration date of the ion selective electrode having the shortest remaining period before the expiration date from among the plural kinds of ion selective electrodes provided in the analysis tank.

3. The automatic analysis device according to Claim 1,
wherein when the use ratio is assumed to be equal in the plurality of the analysis tanks usable for the analysis request and a difference in the target number of times is larger than or equal to a threshold value, the use ratio being determined on the basis of the ratio of the target number of times, the control computer changes the use ratio of the analysis tank such that the analysis tank with the large target number of times is preferentially used.

4. The automatic analysis device according to Claim 1,
wherein among the plurality of analysis tanks, the control computer excludes the analysis tank including the ion selective electrode with the remaining period longer than or equal to the predetermined period from the analysis tanks usable for the analysis request.

5. The automatic analysis device according to Claim 1,
wherein among the plurality of analysis tanks, the control computer excludes the analysis tanks other than the analysis tank specified by a user from the analysis tanks usable for the analysis request.

6. The automatic analysis device according to Claim 5,
wherein the control computer has an analysis priority mode in which the number of analyses is maximized in the predetermined period and a consumable effective use mode, and in the consumable effective use mode, the control computer calculates, for each of the analysis tanks, the target number of times on the basis of the number of remaining possible measurements of the ion selective electrode and a remaining period before the expiration date, and determines, when the plurality of analysis tanks are usable for the analysis request, the use ratio of the analysis tank on the basis of the ratio of the target number of times of the analysis tanks usable for the analysis request.

7. The automatic analysis device according to Claim 6,
wherein a time period is specifiable as the consumable effective use mode.

8. The automatic analysis device according to Claim 1,
wherein when the automatic analysis device starts, the control computer calculates, for each of the analysis tanks, the target number of times on the basis of the number of remaining possible measurements of the ion selective electrode and the remaining period before the expiration date, and determines, when the plurality of analysis tanks are usable for the analysis request, the use ratio of the analysis tank on the basis of the ratio of the target number of times of the analysis tanks usable for the analysis request.

9. A method for allocating an analysis tank to be used for an analysis request, in an automatic analysis device having a plurality of analysis tanks, each including an ion selective electrode for measuring an electrolyte concentration of a sample, and a control computer that allocates the analysis tank to be used for the analysis request, the method comprising the steps of:
causing the control computer to calculate, for each of the analysis tanks, a target number of times the analysis tank is to be used in a predetermined period on a basis of the number of remaining possible measurements of the ion selective electrode and a remaining period before an expiration date,
causing the control computer to determine, when the plurality of analysis tanks are usable for the analysis request, a use ratio of the analysis tank on a basis of a ratio of the target number of times of the analysis tanks usable for the analysis request, and
causing the control computer to allocate the analysis tank to be used for the analysis request according to the use ratio.

10. The method for allocating an analysis tank according to Claim 9,
wherein when the use ratio is assumed to be equal in the plurality of the analysis tanks usable for the analysis request and a difference in the target number of times is larger than or equal to a threshold value, the use ratio being determined on the basis of the ratio of the target number of times, the control computer changes the use ratio of the analysis tank such that the analysis tank with the large target number of times is preferentially used.

11. The method for allocating an analysis tank according to Claim 9,
wherein among the plurality of analysis tanks, the control computer excludes the analysis tank including the ion selective electrode with the remaining period longer than or equal to the predetermined period from the analysis tanks usable for the analysis request.

12. The method for allocating an analysis tank according to Claim 9,
wherein among the plurality of analysis tanks, the control computer excludes the analysis tanks other than the analysis tank specified by a user from the analysis tanks usable for the analysis request.
